# EUROPEAN PATENT SPECIFICATION

(11) **EP 3 958 934 B1**
(45) Date of publication and mention of the grant of the patent: **09.04.2025**
(21) Application number: 20725324.6
(22) Date of filing: 22.04.2020
(51) Int. Cl.: A61M 5/31, A61M 5/50, A61M 39/16

(54) **SYRINGE STERILIZATION VERIFICATION ASSEMBLIES AND METHODS**
ANORDNUNGEN UND VERFAHREN ZUR ÜBERPRÜFUNG DER STERILISATION VON SPRITZEN
ENSEMBLES ET PROCÉDÉS DE VÉRIFICATION DE STÉRILISATION DE SERINGUE

(30) Priority: 24.04.2019 US 201962838087 P
(43) Date of publication of application: 02.03.2022
(73) Proprietor: Amgen Inc., Thousand Oaks, CA 91320-1799 (US)
(72) Inventor: CHOU, Cheng-Chieh, Thousand Oaks, CA 91320-1799 (US); EU, Mingda, Thousand Oaks, CA 91320-1799 (US); FORNG, Ren-Yo, Thousand Oaks, CA 91320-1799 (US); GUIRGUIS, Malak, Thousand Oaks, CA 91320-1799 (US); HENDERSON, Olivia, Alice, Thousand Oaks, CA 91320-1799 (US); LIU, Jessica, Hai, Thousand Oaks, CA 91320-1799 (US); SWIFT, Robert, W., Thousand Oaks, CA 91320-1799 (US); WU, Chia-Jung, Thousand Oaks, CA 91320-1799 (US)
(74) Representative: J A Kemp LLP
(86) International application number: PCT/US2020/029204
(87) International publication number: WO 2020/219482

(56) References cited:
- EP-A1- 0 863 994
- EP-A1- 1 192 965
- EP-B1- 0 863 994
- EP-B1- 1 192 965
- WO-A1-2016/145206
- JP-A- 2006 016 053
- US-A- 5 922 592
- US-A1- 2016 262 984

## Description

### FIELD OF DISCLOSURE

The present disclosure relates generally to containers for pharmaceutical products and, more particularly, providing for the sterility of such containers.

### BACKGROUND

In some forms, a syringe may be provided with separate components that may be assembled as needed for particular tasks. One such syringe includes a barrel having an open distal tip. A mount or adaptor may be secured to the tip of the barrel and provide a structure for removably attaching a cap or needle. Further, the cap can include an elastomeric insert that sealingly engages the tip to create a sterile barrier. EP1192965A1 discloses an example of a syringe having a tip cap.

Syringes such as these can be subjected to a sterilization process with the cap secured to the barrel. JP2006/016053A discloses a method for producing a sterilized packaged medical article. US2016/262984A1 discloses aseptic vial piercing and sterilization systems. EP0863994A1 discloses a sterility indicator. In certain instances, it is necessary to confirm that the interface between the cap and barrel tip is sterile after a sterilization process. Accordingly, one or more of the syringes from a batch may be subjected to a sterilization verification or validation process to ensure that the sterilization process was successful. One such verification process involves positioning a long, flat, rectangular biological indicator (BI) strip between the barrel tip and the elastomeric insert prior to the sterilization process. After the sterilization process is complete, the BI strip can be tested to ensure that the strip has been fully sterilized or otherwise sterilized to sufficient degree by the process. The geometry or configuration of conventional BI strips is such that it may create an open channel between the elastomeric insert and the barrel tip, which tends to skew sterilization verification results.

The present disclosure sets forth sterilization verification methods and assemblies embodying advantageous alternatives to existing sterilization verification methods and assemblies, and that may address one or more of the challenges or needs mentioned above, as well as provide other benefits and advantages.

### SUMMARY

An aspect of the disclosure includes a syringe sterilization verification assembly including a syringe, a tip cap assembly, and a biological indicator (BI) member. The syringe includes a barrel having a tip. The tip cap assembly includes an insert having a skirt configured to receive the tip. The biological indicator (BI) member has an annular portion disposed around at least a portion of the tip and disposed radially between the tip and the skirt of the insert.

In some versions, the annular portion of the BI member comprises a wire member wound in a spiral configuration.

In some versions, the annular portion of the BI member has an elongated sleeve-like shape with a frusto-conical configuration complementary to a configuration of the tapered tip of the barrel.

In some versions, the tip of the barrel defines a channel receiving the BI member.

In some versions, a proximal end of the BI member extends to a location external to a connection between the skirt and the tapered tip of the barrel such that the proximal end of the BI member is not disposed radially between the tapered tip and the skirt of the insert.

In some versions, the BI member extends 360 degrees around the tip of the barrel.

In some versions, the BI member comprises an axial dimension that is less than an axial dimension of the tip.

In some versions, the BI member comprises filter paper carrying bacteria spores.

In some versions, the bacteria spores are generally uniformly dispersed across the entire surface of the BI member.

In some versions, the bacteria spores comprise Bacillus atrophaeus (BA) bacterial spores and/or Geobacillus stearothermophilus (GST) bacterial spores.

Another aspect of the disclosure provides a method for verifying sterilization. The method includes providing a syringe including a barrel and a tip. The method further includes mounting a tip cap assembly on the tip. The method further includes disposing an annular portion of a biological indicator (BI) member around the tapered tip. The method also includes mounting a tip cap assembly having an insert with a skirt on at least a portion of the tip, the skirt having a cavity sized to receive the tip therein, wherein mounting the tip cap assembly on the tip causes the annular portion of the BI member to be disposed radially between the skirt of the insert and the tip. And the method also includes subjecting the syringe, the tip cap assembly, and the BI member to a sterilization process. Finally, the method can include testing the BI member for sterilization.

In some versions, disposing the annular portion of the BI member around at least a portion of the tip comprises wrapping a wire around the tip in a spiral configuration.

In some versions, disposing the annular portion of the BI member around at least a portion of the tip comprises disposing a BI member having an elongated sleeve-like shape with a frusto-conical configuration on the tip.

In some versions, the method also includes disposing the BI member into a channel on the tip.

In some versions, the method also includes disassembling the tip cap assembly and the syringe to access the BI member prior to testing the BI member for sterilization.

In some versions, testing the BI member for sterilization comprises exposing the BI member to media to check for bacterial growth.

Yet another aspect of the disclosure includes a method for verifying sterilization that includes providing a syringe including a barrel and a tip. The method also includes mounting a tip cap assembly on the tip, the tip cap assembly having an insert with a skirt having a cavity to receive the tip therein. The method also includes inoculating an interface between the insert and the tip. The method also includes subjecting the syringe and tip cap assembly to a sterilization process. The method also includes testing the syringe and tip cap assembly for sterilization.

In some versions, inoculating the interface between the insert and the tip comprises penetrating through the insert with a needle and dispensing an inoculation medium between the insert and the tip.

In some versions, the method also includes aseptically disassembling the tip cap assembly and the syringe.

In some versions, testing the syringe and tip cap assembly for sterilization comprises exposing the syringe and tip cap assembly to media to check for bacterial growth.

In accordance with a further aspect, a syringe sterilization verification assembly is disclosed that includes a syringe having a barrel with a tapered tip and a tip cap assembly including an insert with a skirt configured to receive and sealingly engage the tapered tip. The assembly further includes a biological indicator (BI) member that has an annular portion disposed around the tapered tip radially between the tapered tip and the skirt of the insert.

According to some forms, the assembly can include one or more of the following aspects: the annular portion of the BI member is a wire member having a spiral configuration; the annular portion of the BI member has a planar disk-like shape in at least an initial configuration prior to being disposed radially between the tapered tip and the skirt of the insert; the annular portion of the BI member has an elongated sleeve-like shape with a frusto-conical configuration complementary to a configuration of the tapered tip of the barrel; or a proximal end of the BI member can extend to a location external to a connection between the skirt and the tapered tip of the barrel such that the proximal end of the BI member is not disposed radially between the tapered tip and the skirt of the insert.

In accordance with another aspect, a method for verifying sterilization is disclosed that includes providing a syringe including a barrel, a tapered tip, and a Luer collar with a threaded surface, mounting a tip cap assembly on the tapered tip; disposing an annular portion of a biological indicator (BI) member around the tapered tip, and mounting a tip cap assembly having an insert with a skirt on the tapered tip by engaging a threaded surface of the tip cap assembly with the threaded surface of the Luer collar. The skirt has a cavity sized to sealingly receive the tapered tip therein and mounting the tip cap assembly on the tapered tip causes the annular portion of the BI member to be disposed radially between the skirt of the insert and the tapered tip. The method further includes subjecting the syringe, the tip cap assembly, and the BI member to a sterilization technique and testing the BI member for sterilization.

According to some forms, the method can include one or more of the following aspects: disposing the annular portion of the BI member around the tapered tip can include wrapping a wire around the tapered tip in a spiral configuration; the annular portion of the BI member has a planar disk-like shape at least prior to being disposed around the tip of the barrel; or the method can include disassembling the tip cap assembly and the syringe to access the BI member.

In accordance with another aspect, a syringe sterilization verification assembly is disclosed that includes a syringe having a barrel with a tapered tip , wherein a channel is formed in an exterior surface of the tapered tip, a tip cap assembly including an insert having a skirt configured to receive and sealingly engage the tapered tip, and a biological indicator (BI) member disposed in the channel between the tapered tip and the skirt of the insert.

According to some forms, the BI member can be an elongate strip and/or the channel can have an annular configuration extending around the tapered tip.

In accordance with another aspect, a method for verifying sterilization is disclosed that includes providing a syringe including a barrel, a tapered tip, and a Luer collar with a threaded surface, disposing a biological indicator (BI) member in a channel formed in an exterior surface of the tapered tip, and mounting a tip cap assembly having an insert with a skirt on the tapered tip by engaging a threaded surface of the tip cap assembly with the threaded surface of the Luer collar. The skirt has a cavity sized to sealingly receive the tapered tip therein and mounting the tip cap assembly on the tapered tip causes the annular portion of the BI member to be disposed radially between the skirt of the insert and the tapered tip. The method further includes subjecting the syringe, the tip cap assembly, and the BI member to a sterilization technique and testing the BI member for sterilization.

According to some forms, the method can include one or more of the following aspects: disposing the BI member in the channel can include disposing the BI member within an annular channel extending around the tapered tip; the method can include forming the channel in the tapered tip by laser etching; or the method can include disassembling the tip cap assembly and the syringe to access the BI member.

In accordance with another aspect, a method for verifying sterilization is disclosed that includes providing a syringe including a barrel, a tapered tip, and a Luer collar with a threaded surface, mounting a tip cap assembly on the tapered tip, where the tip cap assembly has a threaded surface mating with the threaded surface of the Luer collar and has an insert with a skirt having a cavity to sealingly receive the tapered tip therein. The method further includes inoculating an interface between the insert and the tapered tip, subjecting the syringe and tip cap assembly to a sterilization technique, and testing the syringe and tip cap assembly for sterilization.

According to some forms, the method can include one or more of the following aspects: inoculating the interface between the insert and the tapered tip can include penetrating through the insert with a needle and dispensing an inoculation medium between the insert and the tapered tip; the method can include aseptically disassembling the tapered tip cap assembly and the syringe; or testing the syringe and tip cap assembly for sterilization can include exposing the syringe and tip cap assembly to media to check for growth.

### BRIEF DESCRIPTION OF THE DRAWINGS

Figure 1 is a side elevation view of a syringe assembly in accordance with the present disclosure;
Figure 2 is a cross-sectional view of a first example syringe sterilization verification assembly in accordance with the present disclosure;
Figure 3 is a perspective view of a biological indicator member for the syringe sterilization verification assembly of Figure 2;
Figure 4 is a cross-sectional view of a second example syringe sterilization verification assembly in accordance with the present disclosure;
Figure 5 is a perspective view of a biological indicator member for the syringe sterilization verification assembly of Figure 4;
Figure 6 is a cross-sectional view of a third example syringe sterilization verification assembly in accordance with the present disclosure;
Figure 7 is an exploded view of a biological indicator member and syringe tip for the syringe sterilization verification assembly of Figure 6; and
Figure 8 is a cross-sectional view of a fourth example syringe sterilization verification assembly in accordance with the present disclosure.

### DETAILED DESCRIPTION

Syringe sterilization verification assemblies and methods are described herein that provide consistently reliable results. In some forms, the assemblies and methods utilize biological indicator (BI) members having an annular portion to extend around a tip of a syringe barrel. In alternative or additional forms, the tip of the syringe barrel can include a channel sized to receive the BI member therein. Other forms can utilize a direct inoculation of an interface between a tip cap assembly insert and the tip of the syringe barrel. The BI members disclosed herein may include any conventional or novel materials/compositions and may include, for example, filter paper carrying bacteria spores. In some cases the bacteria spores can include Bacillus atrophaeus (BA) spores and/or in some versions Geobacillus stearothermophilus (GST) bacterial spores. Other spores may be possible. Such BI members can assist with sterility assurance programs by being placed in exposure to the sterilization process, subsequent to which the BI members can be removed, incubated, and tested to determine if any of the bacteria spores survived. In some versions, the bacteria spores are uniformly dispersed across the entire exposed surfaces of the BI members disclosed herein. in other versions, the bacteria spores may be disperse across less than the entire exposed surfaces.

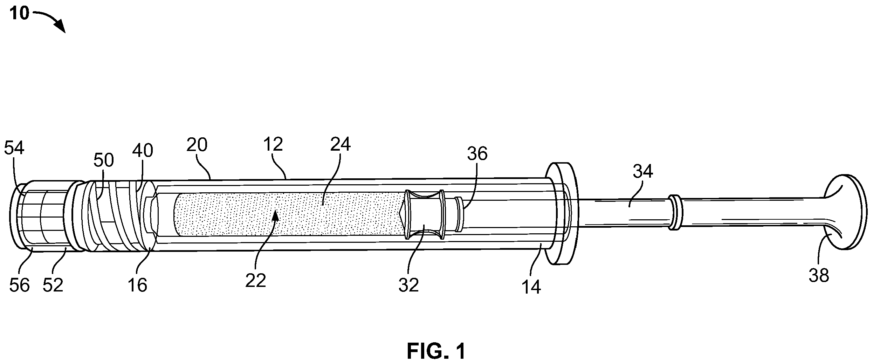

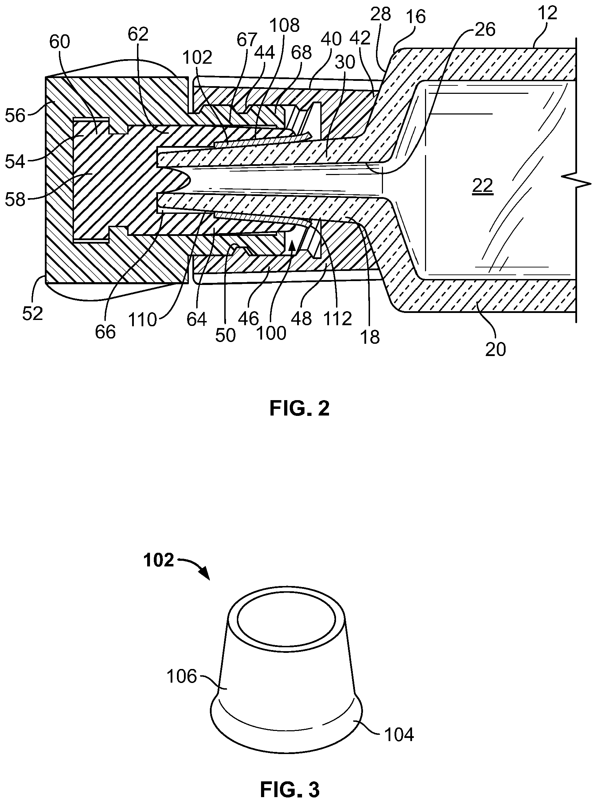

Turning now to Figs. 1 and 2, a hypodermic syringe 10, which in certain embodiments may be configured as a Luer lock syringe, is shown that includes a barrel 12 formed from any suitable material, such as a glass or a polymer. The barrel 12 includes a proximal end 14, a distal end 16 having a tip 18 (shown in Fig. 2), and a cylindrical sidewall 20 extending between the proximal and distal ends 14, 16. The tip 18 and sidewall 20 define a reservoir 22 to receive a therapeutic product 24. The tip 18 further defines a passageway 26 (shown in Fig. 2) fluidly connected to the reservoir 22. With continued reference to Fig. 2, the distal end 16 of the barrel 12 includes a rear portion 28 that has a frusto-conical configuration transitioning to the tip 18. The tip 18 has a frusto-conical sidewall 30 having a predetermined taper. A plunger-stopper 32 is disposed within the reservoir 22 in a sliding, fluid-tight engagement with the sidewall 20. As shown in Fig. 1, the syringe 10 can further include a plunger rod 34 configured to engage the plunger-stopper 32 and drive the plunger-stopper 32 through the barrel 12 to thereby dispense the therapeutic product 24 through the tip 18. The plunger rod 34 can be integrally formed with the plunger-stopper 32 or can have a distal end 36 sized to engage the barrel sidewall 20 and retain the plunger rod 34 within the barrel 12. The plunger rod 34 can include a proximal platform 38 so that a user can conveniently push the plunger rod 34 through the barrel 12. Alternatively, the syringe 10 can be configured for an automatic drug delivery device having a plunger rod and associated drive mechanism such as an on-body injector or autoinjector.

As shown in the embodiments of Fig. 2, the syringe 10 further includes a Luer collar 40 to provide a mount for components of the syringe 10. In a first embodiment, the Luer collar 40 can be integral with the barrel 10 and extend forward from the rear portion 28 of the distal end 16 thereof. The Luer collar 40 has a tubular configuration sized to extend around the tip 18 of the syringe barrel 12. The Luer collar 40 has proximal and distal ends 42, 44 and a sidewall 46 extending therebetween. The proximal end 42 of the collar 40 can include an inwardly projecting portion 48 that extends to the tip 18 of the barrel 12. In a second embodiment, the Luer collar 40 can be attached to the barrel 10. Pursuant to this, the inwardly projecting portion 48 can be an array of inwardly projecting radial protrusions that are sized to fictionally engage the tip 18 of the barrel 12. In an alternative form, the inwardly projecting portion 48 can have an annular configuration. So configured, the collar 40 can be press fit onto the tip 18 by moving the collar 40 in a proximal direction over the tip 18. The inwardly projecting portion 48 can be configured to deflect as the collar 40 is press fit onto the tip 18 so that the collar 40 is frictionally retained on the tip 18. The sidewall 46 has an internally threaded surface 50 to threadingly receive other components of the syringe 10 and mount the other components to the barrel 12.

The syringe 10 further includes a tip cap assembly 52 that secures to the collar 40 to seal to the barrel tip 18. The tip cap assembly 52 includes an insert 54 and a cap 56. The insert 54 includes a head 58 at a distal end 60 thereof and a skirt 62 extending outwardly from the head 58 at a proximal end 64. The skirt 62 defines an interior cavity 66 sized to sealingly engage the barrel tip 18 and the cap 56 includes a sidewall 67 having an exteriorly threaded surface 68 that are configured to engage the threaded surface 50 of the collar 40 to thereby secure the tip cap assembly 52 to the collar 40 and to the barrel 12. The insert 54 is made of an elastomeric material such that the skirt 62 can resiliently expand over and sealingly engage the tip 18. It will be understood that in addition to the tip cap assembly 52, the Luer collar 40 can be utilized to attach a needle assembly for an injection operation.

A first example syringe sterilization verification assembly 100 is shown in Figs. 2 and 3. The assembly 100 includes a BI member 102. The BI member 102 has a body 104 with at least a portion 106 having an annular configuration sized and configured to be disposed around the tip 18 of the barrel 12. In one example, the body 104 has a planar disk-like shape. In some versions, the body 104 has an annular sleeve shape with a continuous, unbroken exterior surface 108. In one form, the body 104 can be formed from a material that is at least partially deformable so that the body 104 can be disposed over and tightly engaging the taper of the barrel tip 18 without damage. In another form, the body 104 can have a frusto-conical configuration, as shown in Fig. 3, with a taper generally complementary to the taper of the barrel tip 18 so that the body 104 can be disposed thereon to tightly engage the barrel tip 18.

As shown in Fig. 2, to verify that the syringe barrel 12 and tip cap assembly 52 mounted thereto has been sterilized, at least a portion of the BI member 102 is disposed in an interface 110 between the skirt 62 and the barrel tip 18. In the disclosed version, the BI member 102 extends 360 degrees in an annular configuration within the interface 110, while having an axial dimension that is less than the axial dimension of the interface 110. In some versions, the BI member 102 can have the same axial dimension of the interface 110. This 360 degree annular configuration advantageously ensures that based on subsequent testing that the sterilization process was effective around the entirety of the interface 110, barrel tip 18, and skirt 62. This is in contrast to merely along one linear side of the barrel tip 18 as was previously attainable through the use of conventional BI strips. In one approach, the BI member 102 can be disposed radially between the skirt 62 and the barrel tip 18. This can be achieved by placing the BI member 102 onto the barrel tip 18 and, if the Luer collar 40 is a separate component, subsequently mounting the Luer collar 40 and tip cap assembly 52 to the barrel tip 18 so that at least a portion of the BI member 102 is disposed within the tip/skirt interface 110 radially therebetween. In one example, the skirt 62 can deflect portions of the BI member 102 that project radially outward from the barrel tip 18 as the tip cap assembly 52 is mounted on the barrel tip 18. In another example, in the embodiment of the body 104 having a planar disk-like shape, the BI member 102 can be disposed in a generally planar configuration extending around an end of the skirt 62 between the skirt 62 and the radially inward extending portion 48 of the Luer collar 40. The Luer collar 40 and tip cap assembly 52 can be threadingly engaged together when mounted to the barrel tip 18, the Luer collar 40 can be mounted to the barrel tip 18 and the tip cap assembly 52 can be subsequently coupled to the Luer collar 40 by threading the cap 56 to the Luer collar 40, or the tip cap assembly 52 can be coupled to the integral Luer collar 40. Alternatively, the Luer collar 40 can be mounted to the barrel tip 18 first, followed by the BI member 102 being disposed on the barrel tip 18, and finally the tip cap assembly 52 can be secured to the Luer collar 40 by engaging the threaded surfaces 50, 68 of the Luer collar 40 and cap 56.

If desired, a proximal end 112 of the body 104 can extend out of the tip/skirt interface 110 to extend along the barrel tip 18 toward the rear portion 28 of the distal end 16. The continuous, unbroken exterior surface 108 of the BI member 102 provides a uniform layered structure with the skirt 62 and barrel tip 18 that better ensures a reliable sterilization verification process.

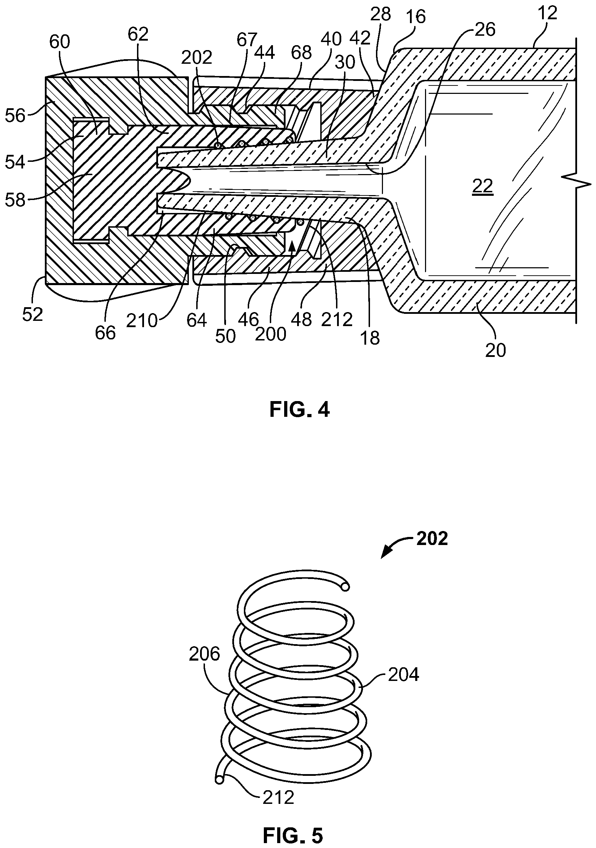

A second example syringe sterilization verification assembly 200 is shown in Figs. 4 and 5. The assembly 200 includes a BI member 202 having a body 204 with at least a portion 206 having an annular configuration sized and configured to be disposed around the tip 18 of the barrel 12. In this example, the body 204 is a wire member that can be wrapped around the barrel tip 18 to create the annular portion 206. Specifically, the wire member 204 can be wrapped along a length of the barrel tip 18 so that the annular portion 206 of the body 204 has a spiral configuration that sequentially increases in diameter to be complementary to the taper of the barrel tip 18. In one form, the BI member 202 can be made of a shape-retentive material so that the body 204 will hold its shape after being wrapped around the barrel tip 18. As with the version described with reference to Figs. 1-3, the BI member 202 of the version in Figs. 4 and 5 extends 360 degrees around the tip 18 of the barrel 12, even if possessing an axial dimension less than an axial dimension of the tip 18. In some versions, the BI member 202 can be coiled additional times around the tip 18 such that the two have the same axial dimension. Regardless, this winding 360 degree configuration advantageously ensures that based on subsequent testing that the sterilization process was effective around the entirety of the tip 18.

As shown in Fig. 4, to verify that the syringe barrel 12 and tip cap assembly 52 mounted thereto has been sterilized, at least a portion of the BI member 202 is disposed in an interface 210 between the skirt 62 and barrel tip 18. This can be achieved by wrapping the BI member 202 around the barrel tip 18 and subsequently mounting the Luer collar 40 and tip cap assembly 52 to the barrel tip 18 so that at least a portion of the BI member 202 is disposed within the tip/skirt interface 210 radially between the skirt 62 and the barrel tip 18. The Luer collar 40 and tip cap assembly 52 can be threadingly engaged together when mounted to the barrel tip 18 or the Luer collar 40 can be mounted to the barrel tip 18 and the tip cap assembly 52 can be subsequently coupled to the Luer collar 40 by threading the cap 56 to the Luer collar 40. Alternatively, the Luer collar 40 can be mounted to the barrel tip 18 first, followed by the BI member 202 being wrapped around the barrel tip 18, and finally the tip cap assembly 52 can be secured to the Luer collar 40 by engaging the threaded surfaces 50, 68 of the Luer collar 40 and cap 56.

If desired, a proximal end 212 of the body 204 can extend out of the tip/skirt interface 210 to extend along the barrel tip 18 toward the rear portion 28 of the distal end 16. The minimal cross-sectional area of the BI member 102 provides a reliable sealing engagement with the skirt 62 and the barrel tip to better ensure a reliable sterilization verification process.

After the syringe 10, tip cap assembly 52, and BI member 102, 202 have been assembled into the sterilization verification assembly 100, 200, the assembly 100, 200 can be subjected to a suitable sterilization process, such as irradiation, chemicals, e.g., ethylene oxide, peracetic acid, ozone, or nitrogen dioxide, heat, pressure, etc. After the assembly 100, 200 has been sterilized, the BI member 102, 202 can be tested to ensure that the sterilization process fully sterilized the skirt/tip interface 110, 210. To test the BI member 102, 202, a user can grip the proximal end 112, 212 and pull the BI member 102, 202 out from the interface 110, 210, particularly with the wire member embodiment. Alternatively, a user can disassemble the tip cap assembly 52 from the Luer collar 40 to expose the BI member 102, 202 for testing. Testing the BI member 102, 202 for sterilization can include exposing the BI member 102, 202 to media to check for growth.

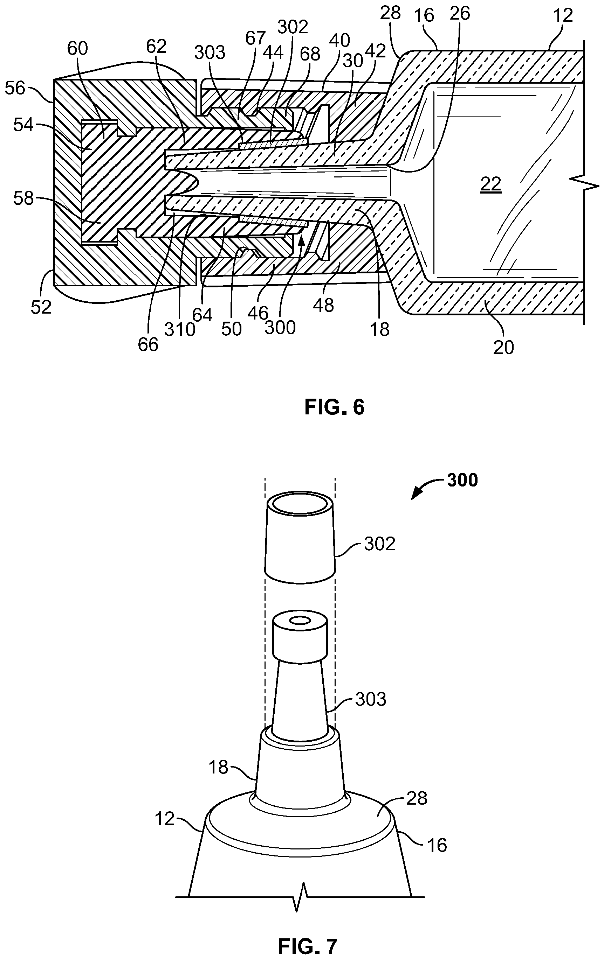

A third example syringe sterilization verification assembly 300 is shown in Figs. 6 and 7. The assembly 300 includes a BI member 302 having a body 304 configured to be disposed 360 degrees around the tip 18 of the barrel 12 in a manner similar to the BI member 102 described with reference to Figs. 1-3 above. Further, the barrel tip 18 can define a recess or channel 303 along a portion thereof reducing the sidewall thickness of the tip 18. The channel 303 can have a depth corresponding to a thickness of the BI member 302 so that with the BI member 302 disposed within the channel 303, the outer surface of the BI member 302 is generally co-planar with the adjacent portions of the barrel tip 18. Further, the BI member 302 and the channel 303 can have complementary dimensions so that the BI member 302 is tightly received therein providing a sealing engagement between the BI member 302 and the bottom and side surfaces of the channel 303. In the illustrated form, the channel 303 has an annular configuration extending around a circumference of the barrel tip 18. Other configurations, such as a spiral configuration, a longitudinal channel extending along a longitudinal length of the barrel tip 18. The BI member 302 can be a strip of suitable material or a ring-shaped member suitable for the particular channel 303 configuration. With the strip and annular channel 303 embodiment, the strip can have a length corresponding to a circumference of the channel 303 so that ends of the strip engage one another when the strip is disposed within the channel 303. The channel 303 can be formed by any suitable method, including, for example, glass forming or etching using any process, such as laser, acid, abrasive blasting, etc. Regardless, as with the previously described embodiments, the BI member 302 of the version in Figs. 6 and 7 extends 360 degrees around the tip 18 of the barrel 12, even if possessing an axial dimension less than an axial dimension of the tip 18. This 360 degree configuration advantageously ensures that based on subsequent testing that the sterilization process was effective around the entirety of the tip 18.

As shown, to verify that the syringe barrel 12 and tip cap assembly 52 mounted thereto has been sterilized, at least a portion of the BI member 302 is disposed in an interface 310 between the skirt 62 and barrel tip 18. This can be achieved by disposing and/or wrapping the BI member 302 within the channel 303 of the barrel tip 18 and subsequently mounting the Luer collar 40 and tip cap assembly 52 to the barrel tip 18 so that at least a portion of the BI member 302 is disposed within the tip/skirt interface 310. The Luer collar 40 and tip cap assembly 52 can be threadingly engaged together when mounted to the barrel tip 18 or the Luer collar 40 can be mounted to the barrel tip 18 and the tip cap assembly 52 can be subsequently coupled to the Luer collar 40 by threading the cap 56 to the Luer collar 40. Alternatively, the Luer collar 40 can be mounted to the barrel tip 18 first, followed by the BI member 302 being disposed within the channel 303 of the barrel tip 18, and finally the tip cap assembly 52 can be secured to the Luer collar 40 by engaging the threaded surfaces 50, 68 of the Luer collar 40 and cap 56.

After the syringe 10, tip cap assembly 52, and BI member 302 have been assembled into the sterilization verification assembly 300, the assembly 300 can be subjected to a suitable sterilization process, such as irradiation, chemicals, e.g., ethylene oxide, peracetic acid, ozone, or nitrogen dioxide, heat, pressure, etc. After the assembly 300 has been sterilized, the BI member 302 can be tested to ensure that the sterilization process fully sterilized the skirt/tip interface 310. To test the BI member 302, a user can disassemble the tip cap assembly 52 from the Luer collar 40 to expose the BI member 302 for testing. Testing the BI member 302 for sterilization can include exposing the BI member 302 to media to check for growth.

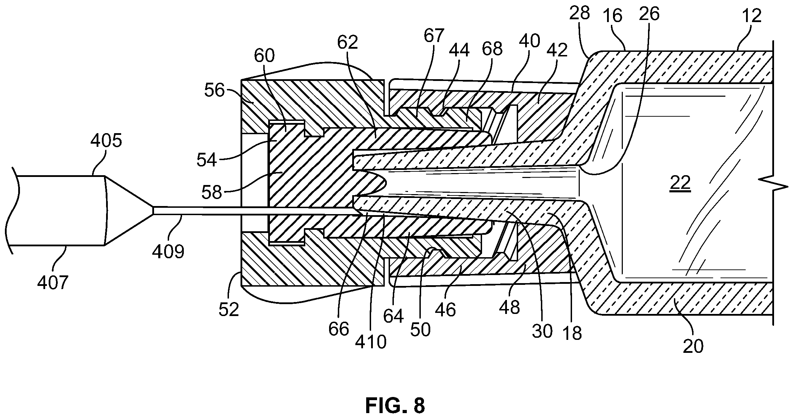

A fourth example syringe sterilization verification assembly 400 is shown in Fig. 8. In this form, the assembly 400 includes an inoculation device 405 that can be utilized to inoculate an interface and/or cavity 410 between the skirt 62 and barrel tip 18. The inoculation device 405 can be a syringe 407 with a needle 409, for example. The needle 309 has a gauge to be inserted through the insert 54 of the tip cap assembly 52 to dispense the inoculation medium and the elastomer of the insert 54 can fully seal the insert opening created by the needle 409. The Luer collar 40 and tip cap assembly 52 can be mounted to the barrel tip 18 in any of the ways described herein. Thereafter, the assembly 400 can be subjected to a suitable sterilization process, such as irradiation, chemicals, e.g., ethylene oxide, peracetic acid, ozone, or nitrogen dioxide, heat, pressure, etc. After the assembly 400 has been sterilized, a user can disassemble the tip cap assembly 52 from the Luer collar 40 and the insert 54 and barrel tip 18 can be tested to ensure that the sterilization process fully sterilized the skirt/tip interface 410. Testing the insert 54 and barrel tip 18 for sterilization can include exposing the insert 54 and/or barrel tip 18 to media to check for growth.

The above description describes various assemblies, devices, and methods for use with a drug delivery device. It should be clear that the assemblies, drug delivery devices, or methods can further comprise use of a drug or medicament listed below with the caveat that the following list should neither be considered to be all inclusive nor limiting. The drug or medicament will be contained in a reservoir. In some instances, the reservoir is a primary container that is either filled or pre-filled for treatment with the drug or medicament. The primary container can be a cartridge or a pre-filled syringe. As used herein, the term drug can be used interchangeably with other similar types of phrases and can be used to mean any type of medicament, therapeutic or non-therapeutic injectable such as traditional and non-traditional pharmaceuticals, nutraceuticals, nutritional supplements, prodrugs (e.g., a compound or molecule which is administered in an inactive or less active state but is cleaved/processed to form the active drug inside the recipient), biologics, biologically active compounds, biologically active molecules, biologically active agents, etc.

For example, the drug delivery device or more specifically the reservoir of the device may be filled with colony stimulating factors, such as granulocyte colony-stimulating factor (G-CSF). Such G-CSF agents include, but are not limited to, Neupogen^{®} (filgrastim) and Neulasta^{®} (pegfilgrastim). In various other embodiments, the drug delivery device may be used with various pharmaceutical products, such as an erythropoiesis stimulating agent (ESA), which may be in a liquid or a lyophilized form. An ESA is any molecule that stimulates erythropoiesis, such as Epogen^{®} (epoetin alfa), Aranesp^{®} (darbepoetin alfa), Dynepo^{®} (epoetin delta), Mircera^{®} (methyoxy polyethylene glycol-epoetin beta), Hematide^{®}, MRK-2578, INS-22, Retacrit^{®} (epoetin zeta), Neorecormon^{®} (epoetin beta), Silapo^{®} (epoetin zeta), Binocrit^{®} (epoetin alfa), epoetin alfa Hexal, Abseamed^{®} (epoetin alfa), Ratioepo^{®} (epoetin theta), Eporatio^{®} (epoetin theta), Biopoin^{®} (epoetin theta), epoetin alfa, epoetin beta, epoetin zeta, epoetin theta, and epoetin delta, as well as the molecules or variants or analogs thereof as disclosed in the following patents or patent applications: U.S. Patent Nos. 4,703,008; 5,441,868; 5,547,933; 5,618,698; 5,621,080; 5,756,349; 5,767,078; 5,773,569; 5,955,422; 5,986,047; 6,583,272; 7,084,245; and 7,271,689; and PCT Publication Nos. WO 91/05867; WO 95/05465; WO 96/40772; WO 00/24893; WO 01/81405; and WO 2007/136752.

An ESA can be an erythropoiesis stimulating protein. As used herein, "erythropoiesis stimulating protein" means any protein that directly or indirectly causes activation of the erythropoietin receptor, for example, by binding to and causing dimerization of the receptor. Erythropoiesis stimulating proteins include erythropoietin and variants, analogs, or derivatives thereof that bind to and activate erythropoietin receptor; antibodies that bind to erythropoietin receptor and activate the receptor; or peptides that bind to and activate erythropoietin receptor. Erythropoiesis stimulating proteins include, but are not limited to, epoetin alfa, epoetin beta, epoetin delta, epoetin omega, epoetin iota, epoetin zeta, and analogs thereof, pegylated erythropoietin, carbamylated erythropoietin, mimetic peptides (including EMP1/hematide), and mimetic antibodies. Exemplary erythropoiesis stimulating proteins include erythropoietin, darbepoetin, erythropoietin agonist variants, and peptides or antibodies that bind and activate erythropoietin receptor (and include compounds reported in U.S. Publication Nos. 2003/0215444 and 2006/0040858) as well as erythropoietin molecules or variants or analogs thereof as disclosed in the following patents or patent applications: U.S. Patent Nos. 4,703,008; 5,441,868; 5,547,933; 5,618,698; 5,621,080; 5,756,349; 5,767,078; 5,773,569; 5,955,422; 5,830,851; 5,856,298; 5,986,047; 6,030,086; 6,310,078; 6,391,633; 6,583,272; 6,586,398; 6,900,292; 6,750,369; 7,030,226; 7,084,245; and 7,217,689; U.S. Publication Nos. 2002/0155998; 2003/0077753; 2003/0082749; 2003/0143202; 2004/0009902; 2004/0071694; 2004/0091961; 2004/0143857; 2004/0157293; 2004/0175379; 2004/0175824; 2004/0229318; 2004/0248815; 2004/0266690; 2005/0019914; 2005/0026834; 2005/0096461; 2005/0107297; 2005/0107591; 2005/0124045; 2005/0124564; 2005/0137329; 2005/0142642; 2005/0143292; 2005/0153879; 2005/0158822; 2005/0158832; 2005/0170457; 2005/0181359; 2005/0181482; 2005/0192211; 2005/0202538; 2005/0227289; 2005/0244409; 2006/0088906; and 2006/0111279; and PCT Publication Nos. WO 91/05867; WO 95/05465; WO 99/66054; WO 00/24893; WO 01/81405; WO 00/61637; WO 01/36489; WO 02/014356; WO 02/19963; WO 02/20034; WO 02/49673; WO 02/085940; WO 03/029291; WO 2003/055526; WO 2003/084477; WO 2003/094858; WO 2004/002417; WO 2004/002424; WO 2004/009627; WO 2004/024761; WO 2004/033651; WO 2004/035603; WO 2004/043382; WO 2004/101600; WO 2004/101606; WO 2004/101611; WO 2004/106373; WO 2004/018667; WO 2005/001025; WO 2005/001136; WO 2005/021579; WO 2005/025606; WO 2005/032460; WO 2005/051327; WO 2005/063808; WO 2005/063809; WO 2005/070451; WO 2005/081687; WO 2005/084711; WO 2005/103076; WO 2005/100403; WO 2005/092369; WO 2006/50959; WO 2006/02646; and WO 2006/29094.

Examples of other pharmaceutical products for use with the device may include, but are not limited to, antibodies such as Vectibix^{®} (panitumumab), Xgeva^{™} (denosumab) and Prolia^{™} (denosamab); other biological agents such as Enbrel^{®} (etanercept, TNF-receptor /Fc fusion protein, TNF blocker), Neulasta^{®} (pegfilgrastim, pegylated filgastrim, pegylated G-CSF, pegylated hu-Met-G-CSF), Neupogen^{®} (filgrastim , G-CSF, hu-MetG-CSF), and Nplate^{®} (romiplostim); small molecule drugs such as Sensipar^{®} (cinacalcet). The device may also be used with a therapeutic antibody, a polypeptide, a protein or other chemical, such as an iron, for example, ferumoxytol, iron dextrans, ferric glyconate, and iron sucrose. The pharmaceutical product may be in liquid form, or reconstituted from lyophilized form.

Among particular illustrative proteins are the specific proteins set forth below, including fusions, fragments, analogs, variants or derivatives thereof:
OPGL specific antibodies, peptibodies, and related proteins, and the like (also referred to as RANKL specific antibodies, peptibodies and the like), including fully humanized and human OPGL specific antibodies, particularly fully humanized monoclonal antibodies, including but not limited to the antibodies described in PCT Publication No. WO 03/002713,, particularly those having the sequences set forth therein, particularly, but not limited to, those denoted therein: 9H7; 18B2; 2D8; 2E11; 16E1; and 22B3, including the OPGL specific antibodies having either the light chain of sequence identification number 2 as set forth therein in Figure 2 and/or the heavy chain of sequence identification number 4, as set forth therein in Figure 4;
Myostatin binding proteins, peptibodies, and related proteins, and the like, including myostatin specific peptibodies, particularly those described in U.S. Publication No. 2004/0181033 and PCT Publication No. WO 2004/058988, particularly in parts pertinent to myostatin specific peptibodies, including but not limited to peptibodies of the mTN8-19 family, including those of sequence identification numbers 305-351, including TN8-19-1 through TN8-19-40, TN8-19 con1 and TN8-19 con2; peptibodies of the mL2 family of sequence identification numbers 357-383; the mL15 family of sequence identification numbers 384-409; the mL17 family of sequence identification numbers 410-438; the mL20 family of sequence identification numbers 439-446; the mL21 family of sequence identification numbers 447-452; the mL24 family of sequence identification numbers 453-454; and those of sequence identification numbers 615-631;
IL-4 receptor specific antibodies, peptibodies, and related proteins, and the like, particularly those that inhibit activities mediated by binding of IL-4 and/or IL-13 to the receptor, including those described in PCT Publication No. WO 2005/047331 or PCT Application No. PCT/US2004/37242 and in U.S. Publication No. 2005/112694, particularly in parts pertinent to IL-4 receptor specific antibodies, particularly such antibodies as are described therein, particularly, and without limitation, those designated therein: L1H1; L1H2; L1H3; L1H4; L1H5; L1H6; L1H7; L1H8; L1H9; L1H10; L1H11; L2H1; L2H2; L2H3; L2H4; L2H5; L2H6; L2H7; L2H8; L2H9; L2H10; L2H11; L2H12; L2H13; L2H14; L3H1; L4H1; L5H1; L6H1;
Interleukin 1-receptor 1 ("IL1-R1") specific antibodies, peptibodies, and related proteins, and the like, including but not limited to those described in U.S. Publication No. 2004/097712, pertinent to IL1-R1 specific binding proteins, monoclonal antibodies in particular, especially, without limitation, those designated therein: 15CA, 26F5, 27F2, 24E12, and 10H7;
Ang2 specific antibodies, peptibodies, and related proteins, and the like, including but not limited to those described in PCT Publication No. WO 03/057134 and U.S. Publication No. 2003/0229023, particularly in parts pertinent to Ang2 specific antibodies and peptibodies and the like, especially those of sequences described therein and including but not limited to: L1(N); L1(N) WT; L1(N) 1K WT; 2xL1(N); 2xL1(N) WT; Con4 (N), Con4 (N) 1K WT, 2xCon4 (N) 1K; L1C; L1C 1K; 2xL1C; Con4C; Con4C 1K; 2xCon4C 1K; Con4-L1 (N); Con4-L1C; TN-12-9 (N); C17 (N); TN8-8(N); TN8-14 (N); Con 1 (N), also including anti-Ang 2 antibodies and formulations such as those described in PCT Publication No. WO 2003/030833, particularly Ab526; Ab528; Ab531; Ab533; Ab535; Ab536; Ab537; Ab540; Ab543; Ab544; Ab545; Ab546; A551; Ab553; Ab555; Ab558; Ab559; Ab565; AbF1AbFD; AbFE; AbFJ; AbFK; AbG1D4; AbGC1E8; AbH1C12; AblA1; AbIF; AblK, AbIP; and AbIP, in their various permutations as described therein;
NGF specific antibodies, peptibodies, and related proteins, and the like including, in particular, but not limited to those described in U.S. Publication No. 2005/0074821 and U.S. Patent No. 6,919,426 particularly as to NGF-specific antibodies and related proteins in this regard, including in particular, but not limited to, the NGF-specific antibodies therein designated 4D4, 4G6, 6H9, 7H2, 14D10 and 14D11;
CD22 specific antibodies, peptibodies, and related proteins, and the like, such as those described in U.S. Patent No. 5,789,554, as to CD22 specific antibodies and related proteins, particularly human CD22 specific antibodies, such as but not limited to humanized and fully human antibodies, including but not limited to humanized and fully human monoclonal antibodies, particularly including but not limited to human CD22 specific IgG antibodies, such as, for instance, a dimer of a human-mouse monoclonal hLL2 gamma-chain disulfide linked to a human-mouse monoclonal hLL2 kappa-chain, including, but limited to, for example, the human CD22 specific fully humanized antibody in Epratuzumab, CAS registry number 501423-23-0;
IGF-1 receptor specific antibodies, peptibodies, and related proteins, and the like, such as those described in PCT Publication No. WO 06/069202, as to IGF-1 receptor specific antibodies and related proteins, including but not limited to the IGF-1 specific antibodies therein designated L1H1, L2H2, L3H3, L4H4, L5H5, L6H6, L7H7, L8H8, L9H9, L10H10, L11H11, L12H12, L13H13, L14H14, L15H15, L16H16, L17H17, L18H18, L19H19, L20H20, L21H21, L22H22, L23H23, L24H24, L25H25, L26H26, L27H27, L28H28, L29H29, L30H30, L31H31, L32H32, L33H33, L34H34, L35H35, L36H36, L37H37, L38H38, L39H39, L40H40, L41H41, L42H42, L43H43, L44H44, L45H45, L46H46, L47H47, L48H48, L49H49, L50H50, L51H51, L52H52, and IGF-1R-binding fragments and derivatives thereof;

Also among non-limiting examples of anti-IGF-1R antibodies for use in the methods and compositions of the present invention are each and all of those described in:
(i) U.S. Publication No. 2006/0040358 (published February 23, 2006), 2005/0008642 (published January 13, 2005), 2004/0228859 (published November 18, 2004), including but not limited to, for instance, antibody 1A (DSMZ Deposit No. DSM ACC 2586), antibody 8 (DSMZ Deposit No. DSM ACC 2589), antibody 23 (DSMZ Deposit No. DSM ACC 2588) and antibody 18 as described therein;
(ii) PCT Publication No. WO 06/138729 (published December 28, 2006) and WO 05/016970 (published February 24, 2005), and Lu et al. (2004), J. Biol. Chem. 279:2856-2865, including but not limited to antibodies 2F8, A12, and IMC-A12 as described therein;
(iii) PCT Publication No. WO 07/012614 (published February 1, 2007), WO 07/000328 (published January 4, 2007), WO 06/013472 (published February 9, 2006), WO 05/058967 (published June 30, 2005), and WO 03/059951 (published July 24, 2003);
(iv) U.S. Publication No. 2005/0084906 (published April 21, 2005), including but not limited to antibody 7C10, chimaeric antibody C7C10, antibody h7C10, antibody 7H2M, chimaeric antibody *7C10, antibody GM 607, humanized antibody 7C10 version 1, humanized antibody 7C10 version 2, humanized antibody 7C10 version 3, and antibody 7H2HM, as described therein;
(v) U.S. Publication Nos. 2005/0249728 (published November 10, 2005), 2005/0186203 (published August 25, 2005), 2004/0265307 (published December 30, 2004), and 2003/0235582 (published December 25, 2003) and Maloney et al. (2003), Cancer Res. 63:5073-5083, including but not limited to antibody EM164, resurfaced EM164, humanized EM164, huEM164 v1.0, huEM164 v1.1, huEM164 v1.2, and huEM164 v1.3 as described therein;
(vi) U.S. Patent No. 7,037,498 (issued May 2, 2006), U.S. Publication Nos. 2005/0244408 (published November 30, 2005) and 2004/0086503 (published May 6, 2004), and Cohen, et al. (2005), Clinical Cancer Res. 11:2063-2073, e.g., antibody CP-751,871, including but not limited to each of the antibodies produced by the hybridomas having the ATCC accession numbers PTA-2792, PTA-2788, PTA-2790, PTA-2791, PTA-2789, PTA-2793, and antibodies 2.12.1, 2.13.2, 2.14.3, 3.1.1, 4.9.2, and 4.17.3, as described therein;
(vii) U.S. Publication Nos. 2005/0136063 (published June 23, 2005) and 2004/0018191 (published January 29, 2004), including but not limited to antibody 19D12 and an antibody comprising a heavy chain encoded by a polynucleotide in plasmid 15H12/19D12 HCA (y4), deposited at the ATCC under number PTA-5214, and a light chain encoded by a polynucleotide in plasmid 15H12/19D12 LCF (κ), deposited at the ATCC under number PTA-5220, as described therein; and
(viii) U.S. Publication No. 2004/0202655 (published October 14, 2004), including but not limited to antibodies PINT-6A1, PINT-7A2, PINT-7A4, PINT-7A5, PINT-7A6, PINT-8A1, PINT-9A2, PINT-11A1, PINT-11A2, PINT-11A3, PINT-11A4, PINT-11A5, PINT-11A7, PINT-11A12, PINT-12A1, PINT-12A2, PINT-12A3, PINT-12A4, and PINT-12A5, as described therein, particularly as to the aforementioned antibodies, peptibodies, and related proteins and the like that target IGF-1 receptors;

B-7 related protein 1 specific antibodies, peptibodies, related proteins and the like ("B7RP-1," also is referred to in the literature as B7H2, ICOSL, B7h, and CD275), particularly B7RP-specific fully human monoclonal IgG2 antibodies, particularly fully human IgG2 monoclonal antibody that binds an epitope in the first immunoglobulin-like domain of B7RP-1, especially those that inhibit the interaction of B7RP-1 with its natural receptor, ICOS, on activated T cells in particular, especially, in all of the foregoing regards, those disclosed in U.S. Publication No. 2008/0166352 and PCT Publication No. WO 07/011941, as to such antibodies and related proteins, including but not limited to antibodies designated therein as follow: 16H (having light chain variable and heavy chain variable sequences designated therein as, respectively, sequence identification number 1 and sequence identification number 7); 5D (having light chain variable and heavy chain variable sequences designated therein as, respectively, sequence identification number 2 and sequence identification number 9); 2H (having light chain variable and heavy chain variable sequences designated therein as, respectively, sequence identification number 3 and sequence identification number 10); 43H (having light chain variable and heavy chain variable sequences designated therein as, respectively, sequence identification number 6 and sequence identification number 14); 41H (having light chain variable and heavy chain variable sequences designated therein as, respectively, sequence identification number 5 and sequence identification number13); and 15H (having light chain variable and heavy chain variable sequences designated therein as, respectively, sequence identification number 4 and sequence identification number 12);
IL-15 specific antibodies, peptibodies, and related proteins, and the like, such as, in particular, humanized monoclonal antibodies, particularly antibodies such as those disclosed in U.S. Publication Nos. 2003/0138421; 2003/023586; and 2004/0071702; and U.S. Patent No. 7,153,507, as to IL-15 specific antibodies and related proteins, including peptibodies, including particularly, for instance, but not limited to, HuMax IL-15 antibodies and related proteins, such as, for instance, 146B7;
IFN gamma specific antibodies, peptibodies, and related proteins and the like, especially human IFN gamma specific antibodies, particularly fully human anti-IFN gamma antibodies, such as, for instance, those described in U.S. Publication No. 2005/0004353, as to IFN gamma specific antibodies, particularly, for example, the antibodies therein designated 1118; 1118*; 1119; 1121; and 1121*. The entire sequences of the heavy and light chains of each of these antibodies, as well as the sequences of their heavy and light chain variable regions and complementarity determining regions, are each individually and specifically disclosed in the foregoing publication and in Thakur et al. (1999), Mol. Immunol. 36:1107-1115. Specific antibodies include those having the heavy chain of sequence identification number 17 and the light chain of sequence identification number 18; those having the heavy chain variable region of sequence identification number 6 and the light chain variable region of sequence identification number 8; those having the heavy chain of sequence identification number 19 and the light chain of sequence identification number 20; those having the heavy chain variable region of sequence identification number 10 and the light chain variable region of sequence identification number 12; those having the heavy chain of sequence identification number 32 and the light chain of sequence identification number 20; those having the heavy chain variable region of sequence identification number 30 and the light chain variable region of sequence identification number 12; those having the heavy chain sequence of sequence identification number 21 and the light chain sequence of sequence identification number 22; those having the heavy chain variable region of sequence identification number 14 and the light chain variable region of sequence identification number 16; those having the heavy chain of sequence identification number 21 and the light chain of sequence identification number 33; and those having the heavy chain variable region of sequence identification number 14 and the light chain variable region of sequence identification number 31, as disclosed in the foregoing publication. A specific antibody contemplated is antibody 1119 as disclosed in the foregoing U.S. publication and having a complete heavy chain of sequence identification number 17 as disclosed therein and having a complete light chain of sequence identification number 18 as disclosed therein;
TALL-1 specific antibodies, peptibodies, and the related proteins, and the like, and other TALL specific binding proteins, such as those described in U.S. Publication Nos. 2003/0195156 and 2006/0135431, as to TALL-1 binding proteins, particularly the molecules of Tables 4 and 5B, each of which is individually and specifically disclosed in the foregoing publications;
Parathyroid hormone ("PTH") specific antibodies, peptibodies, and related proteins, and the like, such as those described in U.S. Patent No. 6,756,480, particularly in parts pertinent to proteins that bind PTH;
Thrombopoietin receptor ("TPO-R") specific antibodies, peptibodies, and related proteins, and the like, such as those described in U.S. Patent No. 6,835,809, particularly in parts pertinent to proteins that bind TPO-R;
Hepatocyte growth factor ("HGF") specific antibodies, peptibodies, and related proteins, and the like, including those that target the HGF/SF:cMet axis (HGF/SF:c-Met), such as the fully human monoclonal antibodies that neutralize hepatocyte growth factor/scatter (HGF/SF) described in U.S. Publication No. 2005/0118643 and PCT Publication No. WO 2005/017107, huL2G7 described in U.S. Patent No. 7,220,410 and OA-5d5 described in U.S. Patent Nos. 5,686,292 and 6,468,529 and in PCT Publication No. WO 96/38557, particularly in parts pertinent to proteins that bind HGF;
TRAIL-R2 specific antibodies, peptibodies, related proteins and the like, such as those described in U.S. Patent No. 7,521,048, particularly in parts pertinent to proteins that bind TRAIL-R2;
Activin A specific antibodies, peptibodies, related proteins, and the like, including but not limited to those described in U.S. Publication No. 2009/0234106, particularly in parts pertinent to proteins that bind Activin A;
TGF-beta specific antibodies, peptibodies, related proteins, and the like, including but not limited to those described in U.S. Patent No. 6,803,453 and U.S. Publication No. 2007/0110747, particularly in parts pertinent to proteins that bind TGF-beta;
Amyloid-beta protein specific antibodies, peptibodies, related proteins, and the like, including but not limited to those described in PCT Publication No. WO 2006/081171, particularly in parts pertinent to proteins that bind amyloid-beta proteins. One antibody contemplated is an antibody having a heavy chain variable region comprising sequence identification number 8 and a light chain variable region having sequence identification number 6 as disclosed in the foregoing publication;
c-Kit specific antibodies, peptibodies, related proteins, and the like, including but not limited to those described in U.S. Publication No. 2007/0253951, particularly in parts pertinent to proteins that bind c-Kit and/or other stem cell factor receptors;
OX40L specific antibodies, peptibodies, related proteins, and the like, including but not limited to those described in U.S. Publication No. 2006/0002929, particularly in parts pertinent to proteins that bind OX40L and/or other ligands of the OX40 receptor; and
Other exemplary proteins, including Activase^{®} (alteplase, tPA); Aranesp^{®} (darbepoetin alfa); Epogen^{®} (epoetin alfa, or erythropoietin); GLP-1, Avonex^{®} (interferon beta-1a); Bexxar^{®} (tositumomab, anti-CD22 monoclonal antibody); Betaseron^{®} (interferon-beta); Campath^{®} (alemtuzumab, anti-CD52 monoclonal antibody); Dynepo^{®} (epoetin delta); Velcade^{®} (bortezomib); MLN0002 (anti- α4β7 mAb); MLN1202 (anti-CCR2 chemokine receptor mAb); Enbrel^{®} (etanercept, TNF-receptor /Fc fusion protein, TNF blocker); Eprex^{®} (epoetin alfa); Erbitux^{®} (cetuximab, anti-EGFR / HER1 / c-ErbB-1); Eylea^{®} or Zaltrap^{®} (aflibercept); Genotropin^{®} (somatropin, Human Growth Hormone); Herceptin^{®} (trastuzumab, anti-HER2/neu (erbB2) receptor mAb); Humatrope^{®} (somatropin, Human Growth Hormone); Humira^{®} (adalimumab); insulin in solution; Infergen^{®} (interferon alfacon-1); Natrecor^{®} (nesiritide; recombinant human B-type natriuretic peptide (hBNP); Kineret^{®} (anakinra); Leukine^{®} (sargamostim, rhuGM-CSF); LymphoCide^{®} (epratuzumab, anti-CD22 mAb); Benlysta^{™} (lymphostat B, belimumab, anti-BlyS mAb); Metalyse^{®} (tenecteplase, t-PA analog); Mircera^{®} (methoxy polyethylene glycol-epoetin beta); Mylotarg^{®} (gemtuzumab ozogamicin); Raptiva^{®} (efalizumab); Cimzia^{®} (certolizumab pegol, CDP 870); Soliris^{™} (eculizumab); pexelizumab (anti-C5 complement); Numax^{®} (MEDI-524); Lucentis^{®} (ranibizumab); Panorex^{®} (17-1A, edrecolomab); Trabio^{®} (lerdelimumab); TheraCim hR3 (nimotuzumab); Omnitarg (pertuzumab, 2C4); Osidem^{®} (IDM-1); OvaRex^{®} (B43.13); Nuvion^{®} (visilizumab); cantuzumab mertansine (huC242-DM1); NeoRecormon^{®} (epoetin beta); Neumega^{®} (oprelvekin, human interleukin-11); Neulasta^{®} (pegylated filgastrim, pegylated G-CSF, pegylated hu-Met-G-CSF); Neupogen^{®} (filgrastim , G-CSF, hu-MetG-CSF); Orthoclone OKT3^{®} (muromonab-CD3, anti-CD3 monoclonal antibody); Procrit^{®} (epoetin alfa); Remicade^{®} (infliximab, anti-TNFα monoclonal antibody); Reopro^{®} (abciximab, anti-GP Ilb/llia receptor monoclonal antibody); Actemra^{®} (anti-IL6 Receptor mAb); Avastin^{®} (bevacizumab), HuMax-CD4 (zanolimumab); Rituxan^{®} (rituximab, anti-CD20 mAb); Tarceva^{®} (erlotinib); Roferon-A^{®}-(interferon alfa-2a); Simulect^{®} (basiliximab); Prexige^{®} (lumiracoxib); Synagis^{®} (palivizumab); 146B7-CHO (anti-IL15 antibody, see U.S. Patent No. 7,153,507); Tysabri^{®} (natalizumab, anti-a4integrin mAb); Valortim^{®} (MDX-1303, anti-B. anthracis protective antigen mAb); ABthrax^{™}; Vectibix^{®} (panitumumab); Xolair^{®} (omalizumab); ETI211 (anti-MRSA mAb); IL-1 trap (the Fc portion of human IgG1 and the extracellular domains of both IL-1 receptor components (the Type I receptor and receptor accessory protein)); VEGF trap (Ig domains of VEGFR1 fused to IgG1 Fc); Zenapax^{®} (daclizumab); Zenapax^{®} (daclizumab, anti-IL-2Rα mAb); Zevalin^{®} (ibritumomab tiuxetan); Zetia^{®} (ezetimibe); Orencia^{®} (atacicept, TACI-Ig); anti-CD80 monoclonal antibody (galiximab); anti-CD23 mAb (lumiliximab); BR2-Fc (huBR3 / huFc fusion protein, soluble BAFF antagonist); CNTO 148 (golimumab, anti-TNFα mAb); HGS-ETR1 (mapatumumab; human anti-TRAIL Receptor-1 mAb); HuMax-CD20 (ocrelizumab, anti-CD20 human mAb); HuMax-EGFR (zalutumumab); M200 (volociximab, anti-α5β1 integrin mAb); MDX-010 (ipilimumab, anti-CTLA-4 mAb and VEGFR-1 (IMC-18F1); anti-BR3 mAb; anti-C. difficile Toxin A and Toxin B C mAbs MDX-066 (CDA-1) and MDX-1388); anti-CD22 dsFv-PE38 conjugates (CAT-3888 and CAT-8015); anti-CD25 mAb (HuMax-TAC); anti-CD3 mAb (NI-0401); adecatumumab; anti-CD30 mAb (MDX-060); MDX-1333 (anti-IFNAR); anti-CD38 mAb (HuMax CD38); anti-CD40L mAb; anti-Cripto mAb; anti-CTGF Idiopathic Pulmonary Fibrosis Phase I Fibrogen (FG-3019); anti-CTLA4 mAb; anti-eotaxin1 mAb (CAT-213); anti-FGF8 mAb; anti-ganglioside GD2 mAb; anti-ganglioside GM2 mAb; anti-GDF-8 human mAb (MYO-029); anti-GM-CSF Receptor mAb (CAM-3001); anti-HepC mAb (HuMax HepC); anti-IFNα mAb (MEDI-545, MDX-1103); anti-IGF1R mAb; anti-IGF-1R mAb (HuMax-Inflam); anti-IL12 mAb (ABT-874); anti-IL12/IL23 mAb (CNTO 1275); anti-IL13 mAb (CAT-354); anti-IL2Ra mAb (HuMax-TAC); anti-IL5 Receptor mAb; anti-integrin receptors mAb (MDX-018, CNTO 95); anti-IP10 Ulcerative Colitis mAb (MDX-1100); anti-LLY antibody; BMS-66513; anti-Mannose Receptor/hCGβ mAb (MDX-1307); anti-mesothelin dsFv-PE38 conjugate (CAT-5001); anti-PD1mAb (MDX-1106 (ONO-4538)); anti-PDGFRα antibody (IMC-3G3); anti-TGFβ mAb (GC-1008); anti-TRAIL Receptor-2 human mAb (HGS-ETR2); anti-TWEAK mAb; anti-VEGFR/Flt-1 mAb; anti-ZP3 mAb (HuMax-ZP3); NVS Antibody #1; and NVS Antibody #2.

Also included can be a sclerostin antibody, such as but not limited to romosozumab, blosozumab, or BPS 804 (Novartis). Further included can be therapeutics such as rilotumumab, bixalomer, trebananib, ganitumab, conatumumab, motesanib diphosphate, brodalumab, vidupiprant, panitumumab, denosumab, NPLATE, PROLIA, VECTIBIX or XGEVA. Additionally, included in the device can be a monoclonal antibody (IgG) that binds human Proprotein Convertase Subtilisin/Kexin Type 9 (PCSK9). Such PCSK9 specific antibodies include, but are not limited to, Repatha^{®} (evolocumab) and Praluent^{®} (alirocumab), as well as molecules, variants, analogs or derivatives thereof as disclosed in the following patents or patent applications: U.S. Patent No. 8,030,547, U.S. Publication No. 2013/0064825, WO2008/057457, WO2008/057458, WO2008/057459, WO2008/063382, WO2008/133647, WO2009/100297, WO2009/100318, WO2011/037791, WO2011/053759, WO2011/053783, WO2008/125623, WO2011/072263, WO2009/055783, WO2012/0544438, WO2010/029513, WO2011/111007, WO2010/077854, WO2012/088313, WO2012/101251, WO2012/101252, WO2012/101253, WO2012/109530, and WO2001/031007.

Also included can be talimogene laherparepvec or another oncolytic HSV for the treatment of melanoma or other cancers. Examples of oncolytic HSV include, but are not limited to talimogene laherparepvec (U.S. Patent Nos. 7,223,593 and 7,537,924); OncoVEXGALV/CD (U.S. Pat. No. 7,981,669); OrienX010 (Lei et al. (2013), World J. Gastroenterol., 19:5138-5143); G207, 1716; NV1020; NV12023; NV1034 and NV1042 (Vargehes et al. (2002), Cancer Gene Ther., 9(12):967-978).

Also included are TIMPs. TIMPs are endogenous tissue inhibitors of metalloproteinases (TIMPs) and are important in many natural processes. TIMP-3 is expressed by various cells or and is present in the extracellular matrix; it inhibits all the major cartilage-degrading metalloproteases, and may play a role in role in many degradative diseases of connective tissue, including rheumatoid arthritis and osteoarthritis, as well as in cancer and cardiovascular conditions. The amino acid sequence of TIMP-3, and the nucleic acid sequence of a DNA that encodes TIMP-3, are disclosed in U.S. Patent No. 6,562,596, issued May 13, 2003. Description of TIMP mutations can be found in U.S. Publication No. 2014/0274874 and PCT Publication No. WO 2014/152012.

Also included are antagonistic antibodies for human calcitonin gene-related peptide (CGRP) receptor and bispecific antibody molecule that target the CGRP receptor and other headache targets. Further information concerning these molecules can be found in PCT Application No. WO 2010/075238.

Additionally, bispecific T cell engager (BiTE^{®}) antibodies, e.g. BLINCYTO^{®} (blinatumomab), can be used in the device. Alternatively, included can be an APJ large molecule agonist e.g., apelin or analogues thereof in the device. Information relating to such molecules can be found in PCT Publication No. WO 2014/099984.

In certain embodiments, the medicament comprises a therapeutically effective amount of an anti-thymic stromal lymphopoietin (TSLP) or TSLP receptor antibody. Examples of anti-TSLP antibodies that may be used in such embodiments include, but are not limited to, those described in U.S. Patent Nos. 7,982,016, and 8,232,372, and U.S. Publication No. 2009/0186022. Examples of anti-TSLP receptor antibodies include, but are not limited to, those described in U.S. Patent No. 8,101,182. In particularly preferred embodiments, the medicament comprises a therapeutically effective amount of the anti-TSLP antibody designated as A5 within U.S. Patent No. 7,982,016.

Although the drug delivery devices, methods, and components thereof, have been described in terms of exemplary embodiments, they are not limited thereto. The detailed description is to be construed as exemplary only and does not describe every possible embodiment of the invention because describing every possible embodiment would be impractical, if not impossible. Numerous alternative embodiments could be implemented, using either current technology or technology developed after the filing date of this patent that would still fall within the scope of the claims defining the invention. For example, components described herein with reference to certain kinds of drug delivery devices, such as on-body injector drug delivery devices or other kinds of drug delivery devices, can also be utilized in other kinds of drug delivery devices, such as autoinjector drug delivery devices.

Those skilled in the art will recognize that a wide variety of modifications, alterations, and combinations can be made with respect to the above described embodiments without departing from the scope of the appended claims, and that such modifications, alterations, and combinations within the scope of the appended claims are to be viewed as being within the invention.

## Claims

1. A syringe sterilization verification assembly comprising:
a syringe (10) including a barrel (12) having a tip (18);
a tip cap assembly (52) including an insert (54) having a skirt (62) configured to receive the tip (18); and
a biological indicator, BI, member (102, 202, 302), **characterized in that** the biological indicator member (102, 202, 302) having an annular portion (106, 206) disposed around at least a portion of the tip (18) and disposed radially between the tip (18) and the skirt (62) of the insert (54).

2. The syringe sterilization verification assembly of claim 1, wherein the annular portion (206) of the BI member (202) comprises a wire member wound in a spiral configuration.

3. The syringe sterilization verification assembly of claim 1, wherein the annular portion (106) of the BI member (102, 302) has an elongated sleeve-like shape with a frusto-conical configuration complementary to a configuration of the tip (18) of the barrel (12).

4. The syringe sterilization verification assembly of claim 3, wherein the tip (18) of the barrel (12) defines a channel (303) receiving the BI member (302).

5. The syringe sterilization verification assembly of any one of the preceding claims, wherein a proximal end of the BI member (102, 202, 302) extends to a location external to a connection between the skirt (62) and the tip (18) of the barrel (12) such that the proximal end of the BI member (102, 202, 302) is not disposed radially between the tip (18) and the skirt (62) of the insert (54).

6. The syringe sterilization verification assembly of any one of the preceding claims, wherein the BI member (102, 202, 302) extends 360 degrees around the tip (18) of the barrel (12).

7. The syringe sterilization verification assembly of any one of the preceding claims, wherein the BI member (102, 202, 302) comprises an axial dimension that is less than an axial dimension of the tip (18).

8. The syringe sterilization verification assembly of any one of the preceding claims, wherein the BI member (102, 202, 302) comprises filter paper carrying bacteria spores, preferably wherein the bacteria spores are generally uniformly dispersed across the entire surface of the BI member (10, 202, 302).

9. The syringe sterilization verification assembly of claim 8, wherein the bacteria spores comprise Bacillus atrophaeus (BA) bacterial spores and/or Geobacillus stearothermophilus (GST) bacterial spores.

10. A method for verifying sterilization, the method comprising:
providing a syringe (10) including a barrel (12) and a tip (18);
disposing an annular portion (106, 206) of a biological indicator ,BI, member (102, 202, 302) around the tip (18);
mounting a tip cap assembly (52) having an insert (54) with a skirt (62) on at least a portion of the tip (18), the skirt (62) having a cavity sized to receive the tip (18) therein, wherein mounting the tip cap assembly (52) on the tip (18) causes the annular portion (106, 206) of the BI member (102, 202, 302) to be disposed radially between the skirt (62) of the insert (54) and the tip (18);
subjecting the syringe (10), the tip cap assembly (52), and the BI member (102, 202, 302) to a sterilization process; and
testing the BI member (102, 202, 302) for sterilization.

11. The method of claim 10, wherein disposing the annular portion (206) of the BI member (202) around at least a portion of the tip (18) comprises wrapping a wire around the tip (18) in a spiral configuration.

12. The method of claim 10, wherein disposing the annular portion (106) of the BI member (102, 302) around at least a portion of the tip (18) comprises disposing a BI member (102, 302) having an elongated sleeve-like shape with a frusto-conical configuration on the tip (18).

13. The method of claim 12, further comprising disposing the BI member (302) into a channel (303) on the tip (18).

14. The method of any one of claims 10-13, further comprising disassembling the tip cap assembly (52) and the syringe (10) to access the BI member (102, 202, 302) prior to testing the BI member (102, 202, 302) for sterilization.

15. The method of any one of claims 10-14, wherein testing the BI member (102, 202, 302) for sterilization comprises exposing the BI member (102, 202, 302) to media to check for bacterial growth.

## Patentansprüche

1. Spritzensterilisations-Verifizierungsanordnung, umfassend:
eine Spritze (10), die einen Zylinder (12) mit einer Spitze (18) enthält;
eine Spitzenkappenanordnung (52), die einen Einsatz (54) mit einer Schürze (62) enthält, die zur Aufnahme der Spitze (18) konfiguriert ist; und
ein biologisches Indikator-, BI-, Element (102, 202, 302), **dadurch gekennzeichnet, dass** das biologische Indikatorelement (102, 202, 302) einen ringförmigen Abschnitt (106, 206) aufweist, der um mindestens einen Abschnitt der Spitze (18) herum und radial zwischen der Spitze (18) und der Schürze (62) des Einsatzes (54) angeordnet ist.

2. Spritzensterilisations-Verifizierungsanordnung nach Anspruch 1, wobei der ringförmige Abschnitt (206) des BI-Elements (202) ein spiralförmig gewundenes Drahtelement umfasst.

3. Spritzensterilisations-Verifizierungsanordnung nach Anspruch 1, wobei der ringförmige Abschnitt (106) des BI-Elements (102, 302) eine längliche hülsenartige Form mit einer kegelstumpfförmigen Konfiguration aufweist, die zu einer Konfiguration der Spitze (18) des Zylinders (12) komplementär ist.

4. Spritzensterilisations-Verifizierungsanordnung nach Anspruch 3, wobei die Spitze (18) des Zylinders (12) einen Kanal (303) definiert, der das BI-Element (302) aufnimmt.

5. Spritzensterilisations-Verifizierungsanordnung nach einem der vorhergehenden Ansprüche, wobei sich ein proximales Ende des BI-Elements (102, 202, 302) zu einer Stelle außerhalb einer Verbindung zwischen der Schürze (62) und der Spitze (18) des Zylinders (12) erstreckt, so dass das proximale Ende des BI-Elements (102, 202, 302) nicht radial zwischen der Spitze (18) und der Schürze (62) des Einsatzes (54) angeordnet ist.

6. Spritzensterilisations-Verifizierungsanordnung nach einem der vorhergehenden Ansprüche, wobei sich das BI-Element (102, 202, 302) um 360 Grad um die Spitze (18) des Zylinders (12) erstreckt.

7. Spritzensterilisations-Verifizierungsanordnung nach einem der vorhergehenden Ansprüche, wobei das BI-Element (102, 202, 302) eine axiale Abmessung umfasst, die geringer ist als eine axiale Abmessung der Spitze (18).

8. Spritzensterilisations-Verifizierungsanordnung nach einem der vorhergehenden Ansprüche, wobei das BI-Element (102, 202, 302) Filterpapier umfasst, das Bakteriensporen trägt, vorzugsweise wobei die Bakteriensporen im Allgemeinen gleichmäßig über die gesamte Oberfläche des BI-Elements (102, 202, 302) verteilt sind.

9. Spritzensterilisations-Verifizierungsanordnung nach Anspruch 8, wobei die Bakteriensporen Bacillus atrophaeus- (BA-) Bakteriensporen und/oder Geobacillus stearothermophilus- (GST-) Bakteriensporen umfassen.

10. Verfahren zur Verifizierung der Sterilisation, wobei das Verfahren umfasst:
Bereitstellen einer Spritze (10), die einen Zylinder (12) und eine Spitze (18) enthält;
Anordnen eines ringförmigen Abschnitts (106, 206) eines biologischen Indikator-, BI-, Elements (102, 202, 302) um die Spitze (18) herum;
Anbringen einer Spitzenkappenanordnung (52) mit einem Einsatz (54) mit einer Schürze (62) auf mindestens einem Abschnitt der Spitze (18), wobei der Schürze (62) einen Hohlraum aufweist, der so bemessen ist, dass er die Spitze (18) darin aufnehmen kann, wobei das Anbringen der Spitzenkappenanordnung (52) an der Spitze (18) bewirkt, dass der ringförmige Abschnitt (106, 206) des BI-Elements (102, 202, 302) radial zwischen der Schürze (62) des Einsatzes (54) und der Spitze (18) angeordnet wird;
Unterziehen der Spritze (10), der Spitzenkappenanordnung (52) und des BI-Elements (102, 202, 302) einem Sterilisationsprozess; und
Prüfen des BI-Elements (102, 202, 302) auf Sterilisation.

11. Verfahren nach Anspruch 10, wobei das Anordnen des ringförmigen Abschnitts (206) des BI-Elements (202) um mindestens einen Abschnitt der Spitze (18) das Wickeln eines Drahtes um die Spitze (18) in einer spiralförmigen Konfiguration umfasst.

12. Verfahren nach Anspruch 10, wobei das Anordnen des ringförmigen Abschnitts (106) des BI-Elements (102, 302) um mindestens einen Abschnitt der Spitze (18) das Anordnen eines BI-Elements (102, 302) mit einer länglichen hülsenartigen Form mit einer kegelstumpfförmigen Konfiguration auf der Spitze (18) umfasst.

13. Verfahren nach Anspruch 12, das ferner das Anordnen des BI-Elements (302) in einem Kanal (303) an der Spitze (18) umfasst.

14. Verfahren nach einem der Ansprüche 10-13, das ferner das Zerlegen der Spitzenkappenanordnung (52) und der Spritze (10) umfasst, um vor dem Testen des BI-Elements (102, 202, 302) auf Sterilisation auf das BI-Element (102, 202, 302) zuzugreifen.

15. Verfahren nach einem der Ansprüche 10-14, wobei das Testen des BI-Elements (102, 202, 302) auf Sterilisation das Aussetzen des BI-Elements (102, 202, 302) gegenüber Medien umfasst, um das Bakterienwachstum zu überprüfen.

## Revendications

1. Ensemble de vérification de stérilisation de seringue, comprenant :
une seringue (10) incluant un corps cylindrique (12) ayant un embout (18) ;
un ensemble capuchon d'embout (52) incluant une pièce d'insertion (54) ayant une jupe (62) configurée pour recevoir l'embout (18) ; et
un élément indicateur biologique, « Biological Indicator » BI, (102, 202, 302), **caractérisé en ce que** l'élément indicateur biologique (102, 202, 302) a une partie annulaire (106, 206) disposée autour d'au moins une partie de l'embout (18) et disposée radialement entre l'embout (18) et la jupe (62) de la pièce d'insertion (54).

2. Ensemble de vérification de stérilisation de seringue de la revendication 1, dans lequel la partie annulaire (206) de l'élément BI (202) comprend un élément fil enroulé en une configuration spirale.

3. Ensemble de vérification de stérilisation de seringue de la revendication 1, dans lequel la partie annulaire (106) de l'élément BI (102, 302) a une forme de type manchon allongé avec une configuration tronconique complémentaire à une configuration de l'embout (18) du corps cylindrique (12).

4. Ensemble de vérification de stérilisation de seringue de la revendication 3, dans lequel l'embout (18) du corps cylindrique (12) définit un canal (303) recevant l'élément BI (302).

5. Ensemble de vérification de stérilisation de seringue de l'une quelconque des revendications précédentes, dans lequel une extrémité proximale de l'élément BI (102, 202, 302) s'étend jusqu'à un emplacement externe à une liaison entre la jupe (62) et l'embout (18) du corps cylindrique (12) de manière telle que l'extrémité proximale de l'élément BI (102, 202, 302) n'est pas disposée radialement entre l'embout (18) et la jupe (62) de la pièce d'insertion (54).

6. Ensemble de vérification de stérilisation de seringue de l'une quelconque des revendications précédentes, dans lequel l'élément BI (102, 202, 302) s'étend sur 360 degrés autour de l'embout (18) du corps cylindrique (12).

7. Ensemble de vérification de stérilisation de seringue de l'une quelconque des revendications précédentes, dans lequel l'élément BI (102, 202, 302) comprend une dimension axiale qui est inférieure à une dimension axiale de l'embout (18).

8. Ensemble de vérification de stérilisation de seringue de l'une quelconque des revendications précédentes, dans lequel l'élément BI (102, 202, 302) comprend un papier-filtre supportant des spores biologiques, de préférence dans lequel les spores biologiques sont dispersées de façon généralement uniforme sur la surface entière de l'élément BI (10, 202, 302).

9. Ensemble de vérification de stérilisation de seringue de la revendication 8, dans lequel les spores biologiques comprennent des spores bactériennes Bacillus atrophaeus (BA) et/ou des spores bactériennes Geobacillus stearothermophilus (GST).

10. Procédé de vérification de stérilisation, le procédé comprenant les faits de :
fournir une seringue (10) incluant un corps cylindrique (12) et un embout (18) ;
disposer une partie annulaire (106, 206) d'un élément indicateur biologique, « Biological Indicator » BI, (102, 202, 302) autour de l'embout (18) ;
monter un ensemble capuchon d'embout (52) ayant une pièce d'insertion (54) avec une jupe (62) sur au moins une partie de l'embout (18), la jupe (62) ayant une cavité dimensionnée pour recevoir l'embout (18) dans celle-ci, dans lequel le fait de monter l'ensemble capuchon d'embout (52) sur l'embout (18) amène la partie annulaire (106, 206) de l'élément BI (102, 202, 302) à être disposée radialement entre la jupe (62) de la pièce d'insertion (54) et l'embout (18) ;
soumettre la seringue (10), l'ensemble capuchon d'embout (52), et l'élément BI (102, 202, 302) à un processus de stérilisation ; et
tester la stérilisation de l'élément BI (102, 202, 302).

11. Procédé de la revendication 10, dans lequel le fait de disposer la partie annulaire (206) de l'élément BI (202) autour d'au moins une partie de l'embout (18) comprend le fait d'enrouler un fil autour de l'embout (18) en une configuration spirale.

12. Procédé de la revendication 10, dans lequel le fait de disposer la partie annulaire (106) de l'élément BI (102, 302) autour d'au moins une partie de l'embout (18) comprend le fait de disposer un élément BI (102, 302) ayant une forme de type manchon allongé avec une configuration tronconique sur l'embout (18).

13. Procédé de la revendication 12, comprenant en outre le fait de disposer l'élément BI (302) dans un canal (303) sur l'embout (18).

14. Procédé de l'une quelconque des revendications 10 à 13, comprenant en outre le fait de désassembler l'ensemble capuchon d'embout (52) et la seringue (10) pour accéder à l'élément BI (102, 202, 302) avant de tester la stérilisation de l'élément BI (102, 202, 302).

15. Procédé de l'une quelconque des revendications 10 à 14, dans lequel le fait de tester la stérilisation de l'élément BI (102, 202, 302) pour comprend le fait d'exposer l'élément BI (102, 202, 302) à des milieux pour contrôler une croissance bactérienne.
